# EUROPÄISCHE PATENTSCHRIFT

(11) **EP 0 582 901 B1**
(45) Veröffentlichungstag und Bekanntmachung des Hinweises auf die Patenterteilung: **03.07.1996**
(21) Anmeldenummer: 93112094.3
(22) Anmeldetag: 29.07.1993
(51) Int. Cl.: C07C 7/13

(54) **Verfahren zur Abtrennung von Acetaldehyd von Kohlenwasserstoffen**
Process for the removal of acetaldehyde from hydrocarbons
Procédé pour l'élimination d'acétaldéhyde d'hydrocarbures

(30) Priorität: 08.08.1992 DE 4226302
(43) Veröffentlichungstag der Anmeldung: 16.02.1994
(73) Patentinhaber: BASF Aktiengesellschaft, D-67063 Ludwigshafen (DE)
(72) Erfinder: Mueller, Ulrich, Dr., D-6730 Neustadt (DE); Weiss, Ralf, D-6700 Ludwigshafen (DE); Diehl, Klaus, Dr., D-6733 Hassloch (DE); Sandrock, Gerhard, Dr., D-6710 Frankenthal (DE); Sauvage, Lothar, D-6700 Ludwigshafen (DE)

(56) Entgegenhaltungen:
- US-A- 3 663 641
- DATABASE WPI Section Ch, Week 8409, Derwent Publications Ltd., London, GB; Class A41, AN 84-054362 & SU-A-1 011 624 (GEOR OIL RES INST) 15. April 1983
- DATABASE WPI Section Ch, Week 8409, Derwent Publications Ltd., London, GB; Class A41, AN 84-054362 & SU-A-1 011 624 (GEOR OIL RES INST) 15. April 1983 & CHEMICAL ABSTRACTS, vol. 99. no. 7, 15. August 1983, Columbus, Ohio, US; abstract no. 53098q, Seite 502;

## Beschreibung

Die vorliegende Erfindung betrifft ein Verfahren zur Abtrennung von Acetaldehyd von C₃-C₁₅-Kohlenwasserstoffen.

Bei der thermischen oder thermisch-katalytischen Spaltung von Erdgas, Raffineriegas oder bestimmten Erdölfraktionen, wie sie z.B. in Steamcrackern durchgeführt wird, fallen nach der Aufarbeitung der Spaltprodukte u.a. Gemische aus Butadien, Butanen, n-Butenen und iso-Buten an, die allgemein als C₄-Schnitte (Weissermel, Arpe, Industrielle Organische Chemie, Verlag Chemie 1988, S. 68) bezeichnet werden.

Diese C₄-Schnitte enthalten Spuren von Acetaldehyd. Wird Butadien aus dem C₄-Schnitt extraktiv entfernt, wird auch Acetaldehyd von den verbleibenden Kohlenwasserstoffen, dem sogenannten Raffinat, abgetrennt. Wählt man zur Weiterverarbeitung des C₄-Schnittes aber z.B. eine selektive Hydrierung von Butadien zu Buten, entfällt die Entfernung des Acetaldehyds. Dieser ist in Kohlenwasserstoffen prinzipiell unerwünscht. Acetaldehyd kann u.a. in den Verfahrensstufen zur Weiterverarbeitung von C₄-Schnitten zur Desaktivierung der entsprechenden Katalysatoren führen.

Die SU-A 1011624 lehrt ein Verfahren zur Abtrennung von Carbonylverunreinigungen aus gasförmigen Isobuten-Strömen durch Adsorption an einen Magnesium-A-Zeolithen (Mg-A-Zeolith).

Die Adsorption von reinem, gasförmigen Acetaldehyd an einen Natrium-A-Zeolithen wird in J. Chem. Soc. Faraday Trans, 86 (1990) 205 im Zusammenhang mit Infrarot-Untersuchungen beschrieben.

Es bestand die Aufgabe, ein Verfahren bereitzustellen, das möglichst selektiv und wirtschaftlich Acetaldehyd von Kohlenwasserstoffen trennt.

Dieser Aufgabe entsprechend wurde ein Verfahren zur Abtrennung von Acetaldehyd von C₃-C₁₅-Kohlenwasserstoffen gefunden, das dadurch gekennzeichnet ist, daß man den Kohlenwasserstoff oder ein Kohlenwasserstoffgemisch in flüssiger Phase mit einem Zeolithen in Kontakt bringt, dessen Porenweite größer als 0,3 nm und bis zu 0,75 nm ist.

Die Adsorption des Acetaldehyds erfolgt an Zeolithen. Zeolithe, auch als Molekularsiebe bezeichnet, sind kristalline Aluminosilikate, die durch eine hochgeordnete Struktur mit einem starren dreidimensionalen Netzwerk von SiO₄- und Al04-Tetraedern gekennzeichnet sind, die durch gemeinsame Sauerstoffatome verbunden sind. Die Elektrovalenz der Aluminium enthaltenden Tetraeder ist durch Einschluß von Kationen in den Kristall, z.B. eines Alkalimetall- oder Wasserstoffions, ausgeglichen. Ein Kationenaustausch ist möglich. In den Zeolithen können anstelle von Aluminium auch andere Elemente wie B, Ga, Fe, Cr, V, As, Sb, Bi oder Be oder ihre Gemische im Gitter enthalten sein und auch das Silicium kann durch ein anderes vierwertiges Element wie Ge, Ti, Zr oder Hf ersetzt sein.

Für das erfindungsgemäße Verfahren kommen Zeolithe mit einer Porenweite von über 0,3 bis 0,75 nm in Betracht. Diese Porenweite ist durch den Kristallaufbau bestimmt und kann z.B. aus Röntgenstrukturdaten bestimmt werden. In Zeolithe mit kleinerer Porenweite kann Acetaldehyd aufgrund seiner räumlichen Ausdehnung nur schlecht hineindiffundieren und somit nicht befriedigend adsorbiert werden. Bevorzugt werden Zeolithe vom A-, L-, X- und vom Y-Typ, wobei A- und X-Typ besonders bevorzugt sind. Ganz besonders bevorzugt sind Natrium-A-Zeolithe.

Na-A-Zeolithe mit einer Porenweite von 0,4 nm sind besonders geeignet, Acetaldehyd von Gemischen aus Propen, Butan, Buten-2 und Butadien abzutrennen. Liegt Acetaldehyd nur im Gemisch mit verzweigten Kohlenwasserstoffen wie Isobutan oder Isobuten vor, ist es besonders vorteilhaft, einen Na-A-Zeolithen zu verwenden oder den Na-A-Zeolithen teilweise oder vollständig durch Erdalkalimetall-A-Zeolithe wie Ca-A-Zeolithe zu ersetzen.

Die Herstellung der Zeolithe erfolgt nach bekannten Methoden (z.B. Ullmanns Encyclopädie d. Techn. Chemie, 4. Aufl., Bd. 17, S. 9-17 (1983)). Die Zeolithe können nach dem Entfernen eingeschlossenen Wassers durch Calcinieren mit Bindemitteln zu Strängen oder Tabletten verformt werden. Als Bindemittel kommen Aluminiumoxide, Siliciumdioxid, Gemische aus hochdispersem SiO₂ mit A1₂O₃, TiO₂, ZrO₂ oder Ton in Betracht.

Das erfindungsgemäße Verfahren erlaubt die Abtrennung von Acetaldehyd von Kohlenwasserstoffen, die 3 bis 15 Kohlenstoffatome haben. Kleinere Kohlenwasserstoffe sind Acetaldehyd in ihrer räumlichen Ausdehnung so ähnlich, daß sie im Zeolithen in Konkurrenz für eine Adsorption treten. Die Kohlenwasserstoffe umfassen Alkane wie Propan, n-Butan, iso-Butan, n-Pentan, 2-Methylbutan, 3-Methylbutan, Alkene wie Propen, 1-Buten, 2-Buten, 1-Penten, Cyclohexen, aber auch 1,3-Butadien, Alkine und aromatische Verbindungen wie Benzol, Toluol, O-Xylol, m-Xylol und p-Xylol. Von besonderer Bedeutung ist das Verfahren zur Reinigung von C₄-Schnitten, aus denen Butadien schon abgetrennt sein kann, und für Iso-buten.

Das erfindungsgemäße Verfahren ist besonders vorteilhaft zur Entfernung von Acetaldehydmengen von 10 - 500 ppm, wie sie etwa in C₄-Schnitten anfallen. Es ist aber auch in der Lage, größere Acetaldehyd-Konzentration abzubauen, die im Bereich von bis zu 50 000 ppm (5 Gew.-%) liegen.

Die Temperatur für das erfindungsgemäße Verfahren liegt in der Regel bei -10 bis 60°C, vorzugsweise 15 bis 35°C. Tiefere Temperaturen sind für große Ströme nur aufwendig zu realisieren, bei höheren läßt das Adsorptionsvermögen der Zeolithe merklich nach.

Der Druck wird so gewählt, daß die zu reinigenden Kohlenwasserstoffe flüssig vorliegen. Der Druck beträgt im allgemeinen 1 bis 70, bevorzugt 5 bis 35 bar. Es ist vorteilhaft, unter demjenigen Druck zu arbeiten, unter dem der Kohlenwasserstoff bei seiner Herstellung, z.B. durch Cracken von Erdöl, flüssig anfällt.

Bei der Reinigung großer Produktströme hat es sich bewährt, den Zeolithen in Form einer Festbettschüttung in einer Adsorptionssäule, durch die der Kohlenwasserstoff geleitet wird, einzusetzen. Diese Säulen werden vom zu reinigenden Kohlenwasserstoff in auf- oder absteigender Fahrweise durchströmt. Die Höhe der Schüttung beträgt hierbei vorzugsweise das 2- bis 15-fache des Durchmessers der Schüttung. Beim Durchströmen empfehlen sich lineare Geschwindigkeiten des Flüssigkeitsquerschnitts, berechnet aus dem Verhältnis von Volumenstrom zu Adsorberbettquerschnitt, von 0,5 bis 35 cm/min, vorzugsweise von 1 bis 15 cm/min und insbesondere von 1,5 bis 10 cm/min.

Ist der Zeolith gesättigt, kann man ihn durch Überleiten eines Inertgases wie Stickstoff bei Normaldruck und Temperaturen von 150 bis 250°C regenerieren, wofür man etwa 4 bis 12 Stunden benötigt.

Für die kontinuierliche Arbeitsweise ist es zweckmäßig, zwei Adsorptionssäulen vorzusehen, von denen jeweils die eine auf die Adsorption und die andere auf die Desorption (Regenerierung) geschaltet wird. Auch mehrere Adsorptionssäulen können nach diesem Prinzip parallel geschaltet werden.

Das erfindungsgemäße Verfahren erlaubt die Abtrennung von Acetaldehyd von C₃-C₁₅-Kohlenwasserstoffen bis zu einem Restgehalt von weniger als 1 ppm. Diese Restgehalte stehen in der Regel einer Verwendung oder Verfahrensschritten zur Weiterverarbeitung der Kohlenwasserstoffe nicht entgegen. Der dazu verwendete Zeolith ist leicht regenerierbar. Weiterhin werden auch Wasserspuren aus den eingesetzten Kohlenwasserstoffen adsorptiv entfernt.

### Beispiele

Kohlenwasserstoffe mit bestimmten Acetaldehyd-Konzentrationen wurden über Zeolithe geleitet. Gemessen wurde die Acetaldehyd-Konzentration nach der Adsorption nach verschiedenen Zeiten. Einzelheiten sind der nachstehenden Tabelle zu entnehmen.

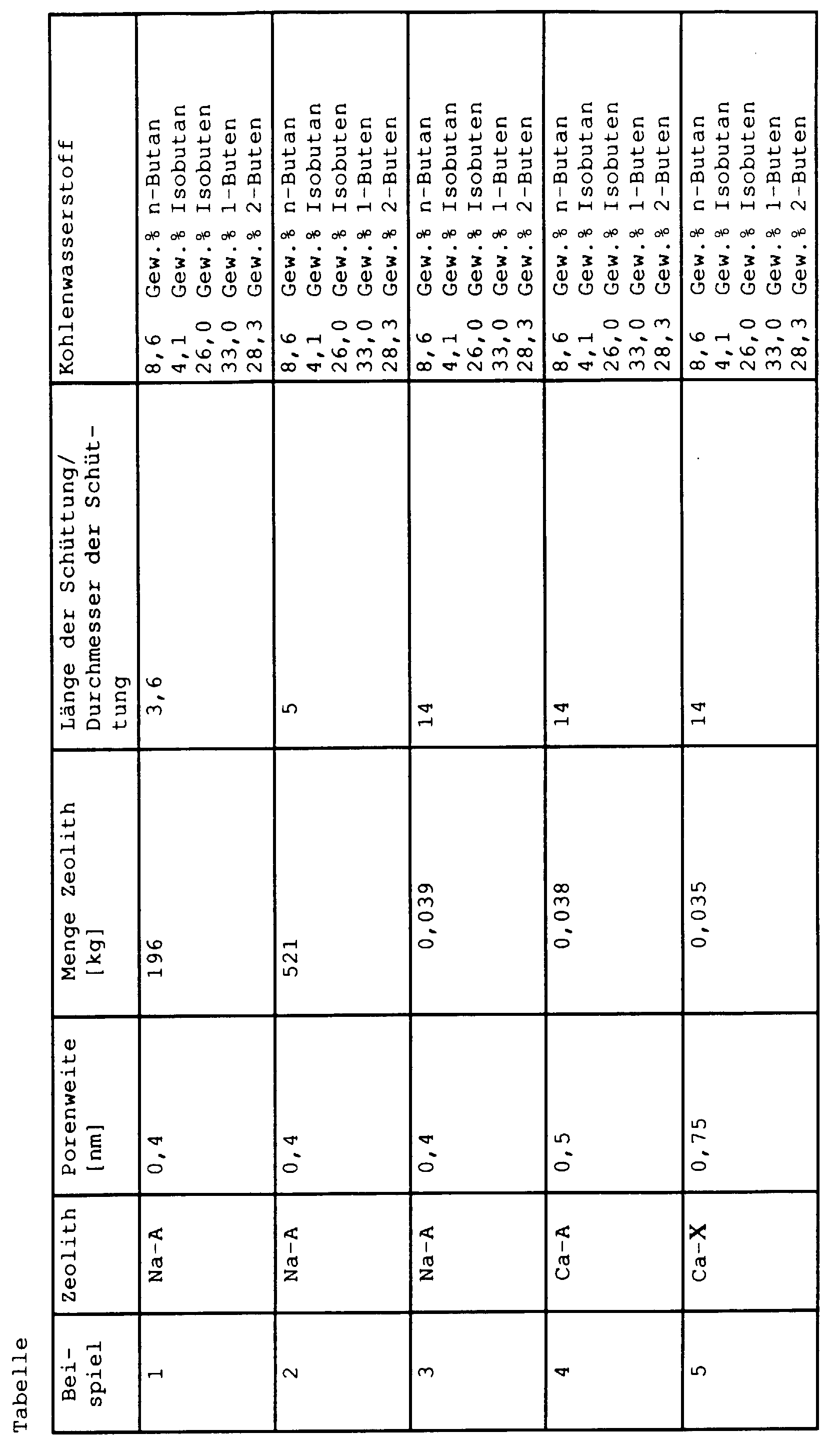

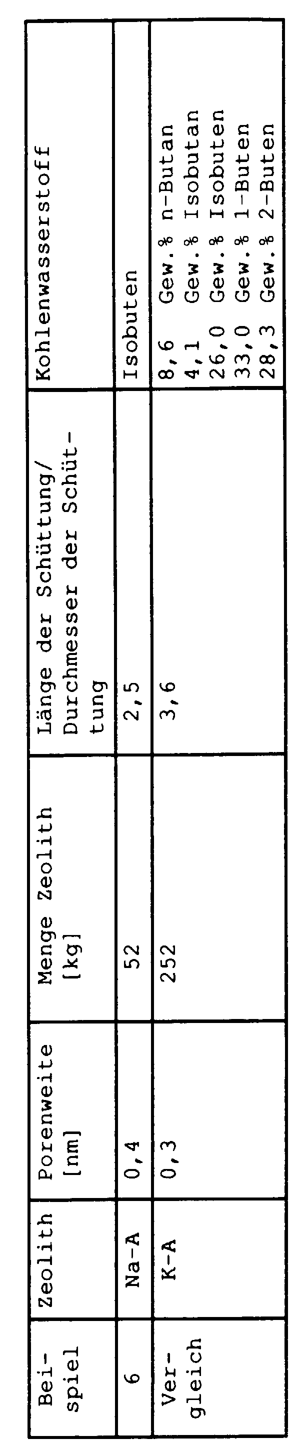

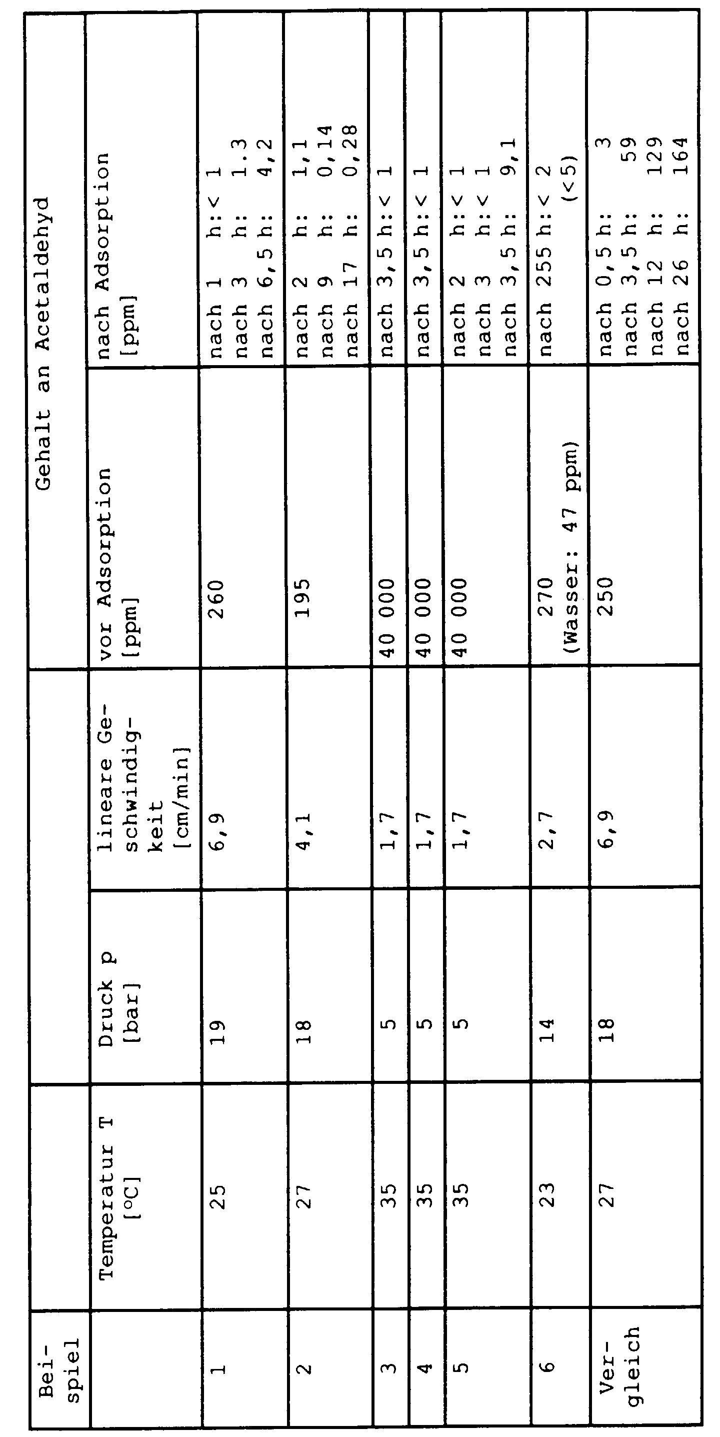

## Patentansprüche

1. Verfahren zur Abtrennung von Acetaldehyd von C₃-C₁₅-Kohlenwasserstoffen, dadurch gekennzeichnet, daß man den Kohlenwasserstoff oder ein Kohlenwasserstoffgemisch in flüssiger Phase mit einem Zeolithen in Kontakt bringt, dessen Porenweite größer als 0,3 nm und bis zu 0,75 nm ist.

2. Verfahren nach Anspruch 1, dadurch gekennzeichnet, daß es sich bei dem Zeolithen um einen A- oder X-Zeolithen handelt.

3. Verfahren nach Anspruch 1 oder 2, dadurch gekennzeichnet, daß man ein Acetaldehyd enthaltendes Gemisch aus Butanen, n-Butenen und iso-Buten einsetzt.

4. Verfahren nach Anspruch 1 oder 2, dadurch gekennzeichnet, daß man Acetaldehyd enthaltendes iso-Buten einsetzt.

## Claims

1. A process for removing acetaldehyde from C₃-C₁₅ hydrocarbons, which comprises contacting the hydrocarbon or a hydrocarbon mixture in liquid phase with a zeolite whose pores have a width which is greater than 0.3 nm and up to 0.75 nm.

2. A process as claimed in claim 1, wherein the zeolite is an A or X zeolite.

3. A process as claimed in claim 1 or 2, wherein an acetaldehyde-containing mixture of butanes, n-butenes and isobutene is employed.

4. A process as claimed in claim 1 or 2, wherein acetaldehyde-containing isobutene is employed.

## Revendications

1. Procédé de séparation de l'acétaldéhyde d'hydrocarbures en C₃ à C₁₅, caractérisé en ce que l'on met l'hydrocarbure, ou un mélange d'hydrocarbures, en phase liquide, en contact avec une zéolite dont la largeur des pores est supérieure à 0,3 nm et s'étend jusqu'à 0,75 nm.

2. Procédé suivant la revendication 1, caractérisé en ce qu'il s'agit dans le cas de la zéolite, d'une zéolite A ou X.

3. Procédé suivant la revendication 1 ou 2, caractérisé en ce que l'on utilise un mélange de butanes, de n-butènes et d'isobutène, contenant de l'acétaldéhyde.

4. Procédé suivant la revendication 1 ou 2, caractérisé en ce que l'on utilise de l'isobutène contenant de l'acétaldéhyde.
